# EUROPEAN PATENT SPECIFICATION

(11) **EP 0 591 362 B1**
(45) Date of publication and mention of the grant of the patent: **13.11.1996**
(21) Application number: 92913764.4
(22) Date of filing: 24.06.1992
(51) Int. Cl.: A61M 5/32

(54) **INCINERATOR, PARTICULARLY FOR USED MEDICAL NEEDLES**
VERBRENNUNGSVORRICHTUNG,INSBESONDERE FUER ZU MEDIZINISCHEN ZWECKEN GEBRAUCHTE NADELN
INCINERATEUR UTILISE EN PARTICULIER POUR DES AIGUILLES MEDICALES

(30) Priority: 25.06.1991 GB 91136515; 24.04.1992 US 874296
(43) Date of publication of application: 13.04.1994
(73) Proprietor: Greville, Peter, Limassol (CY)
(72) Inventor: GREVILLE, Peter, Limassol (CY); SHUREY, Mark, S., Limassol (CY); LAWRENCE, Anthony, H., Limassol (CY)
(74) Representative: Gilding, Martin John
(86) International application number: GB9201149
(87) International publication number: WO9300121

(56) References cited:
- EP-A- 0 374 439
- DE-U- 9 004 788
- US-A- 4 628 169
- US-A- 5 076 178

## Description

The present invention relates to an incinerator, and in particular to an incinerator for incineration (thermal degradation) of electrically-conductive hollow articles such as medical needles.

Incinerators particular adapted for the incineration (thermal degradation) of potentially hazardous used medical needles are disclosed in, for example, US-A-4628169 and South African Patent Specification 89/8607. Both documents disclose incinerators for thermally degrading spent syringe needles using a pair of spaced electrical contacts to pass a high current through an interposed medical needle.

US-A-4628169 describes an incinerator suitable for incinerating a syringe needle including a pair of electrically-conductive contacts arranged to contact a portion of the needle at separate points along the length thereof to thermally degrade said portion when electricity is passed between the pair of contacts through the article to incinerate said portion of the article.

An improved incinerator has now been devised which may be used for such purposes.

By using such an incinerator the portion of the article between the first mentioned pair of contacts is thermally degraded and thus incinerated and a remaining projecting part of the article is plastically deformed and is welded and sealed by the second pair of contacts, due to the passage of a sufficient high current through the respective pairs of contacts.

The first pair of contacts are arranged such that when the hollow article is introduced into the incinerator, the gap between the first pair of contacts is bridged and a current flows through the portion of the article (ideally extending up from the tip of the article) bridging the first pair of contacts. A sufficiently high current flowing through the bridging portion of the article causes that portion of the article to be thermally degraded (incinerated) e.g. by melting and thermal oxidation.

Preferably, the second pair of contacts are arranged to plastically deform a section of the article immediately adjacent the incinerated portion.

Advantageously, where the hollow article is elongate (e.g. a medical needle) the first pair of contacts will be spaced from the second pair of contacts in the longitudinal direction of the article.

Ideally the incinerator comprises means by which a current may be caused to flow through that part of the hollow conductive article between the first and second pairs of contacts causing that part of the hollow article to be incinerated (or pyrolysed). This current may be supplied by a third pair of contacts which comprise one contact from each of said first and second pairs of contacts.

It is preferred that movement means is provided to actuate automatically at a predetermined stage in the incineration process (to cause relative movement towards one another of the second pair of contacts), advantageously being triggered when a predetermined length and gauge of the article has been inserted into the incinerator. Typically, insertion of the article into the incinerator causes a movable plunger to complete a circuit when a predetermined plunger position is reached. Completion of this circuit typically causes an electric motor or the like to actuate the movement means. The plunger may for example contact the actuation arm of a microswitch which actuates a driving motor/lead screw arrangement to cause a movable contact jaw, as one of the contacts, to more relative to a stationary contact jaw, as the other contact, thereby crimping (plastically deforming) the article.

Advantageously, when the article has been sealed, the microprocessor and/or integrated circuitry causes an electric motor or the like to actuate the incineration of the remaining article placed between the first and second pains of contacts. The microprocessor and/or integrated circuitry may for example actuate a driving motor/lead screw arrangement to cause the first pair of contacts to move, and being electrically-conductive thus cause the incineration of the remaining said article.

It is preferred that the timing and duration of operation of the movement means and electrical energisation of the first (and second) contacts is controlled by means of appropriate microprocessor and/or integrated circuitry.

The incinerator is preferably provided with a removable receptacle arranged to collect the incinerated (melted) portions of the article.

Typically, the second pair of contacts, and also preferably the first pair of contacts, are provided in a discrete module separable from the remainder of the incinerator. Where the power supply means is provided in the remainder of the incinerator, the discrete module is advantageously provided with electrical coupling means adapted to cooperatively engage with complementary couplings of the power supply means.

Typically, the power supply means will be a voltage step down transformer provided internally of the housing. Alternatively, rechargeable batteries may be provided in the housing.

The invention will now be further described in a specific embodiment by way of example only and with reference to the accompanying drawings, in which:-
Figure 1 is an external schematic view of an incinerator according to the invention mounted on a stand together with a waste container:
Figure 2 is a perspective view of the incinerator of Figure 1;
Figure 3 is a further perspective view of the incinerator of Figures 1 and 2;
Figure 4 is a schematic view of the internal structure of the incinerator of Figures 1 to 3;
Figure 5 is a further schematic view of the internal structure of the incinerator of Figures 1 to 4;
Figures 6A to 6C are schematic and sectional views of a part of the incinerator of Figures 1 to 5;
Figures 7A to 7C are schematic and sectional views of parts of the incinerator shown in Figures 6A to 6C;
Figure 8 is a sectional schematic view of parts of the incinerator shown in Figures 6A to 7C;
Figures 9A to 9F are schematic and sectional views of parts of the incinerator shown in Figures 6A to 7C showing the sequence of operation of the incinerator;
Figures 10A to 10C are schematic and sectional views of parts of the incinerator shown in Figures 6A to 7C and Figues 9A to 9F.
Figure 11 is a schematic view of the operational electrical circuitry of the incinerator of Figures 1 to 10C;
Figure 12 is a schematic view of a part of the operational electrical circuitry of the incinerator of Figure 11;
Figure 13 is a schematic view of a part of the operational electrical circuitry of the incinerator of Figures 11 and 12;
Figures 14A to 14D are schematic views of parts of the operational electrical circuitry of the incinerator of Figures 11 to 13;
Figure 15 is a schematic view of a part of the operational electrical circuitry of the incinerator of Figures 11 and 14A to 14D.

The incinerator unit, generally designated 80, is typically mounted on a stand 61 which is adapted also to house a general medical waste container 162. The incinerator is particularly adapted for the hygienic destruction of used syringe needles 64 and, since it is portable, the incinerator may conveniently be moved between locations (e.g. in a hospital) where needed.

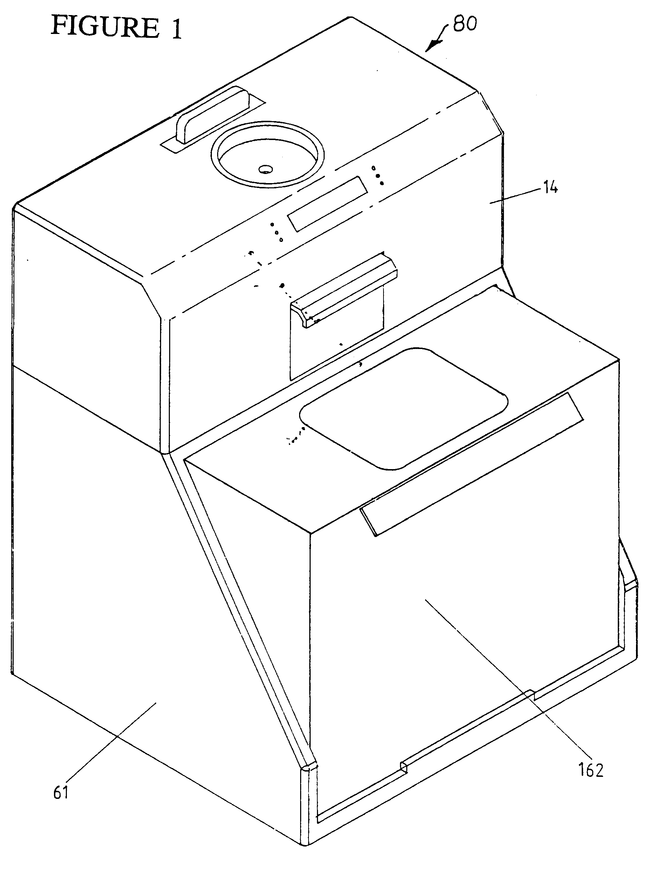

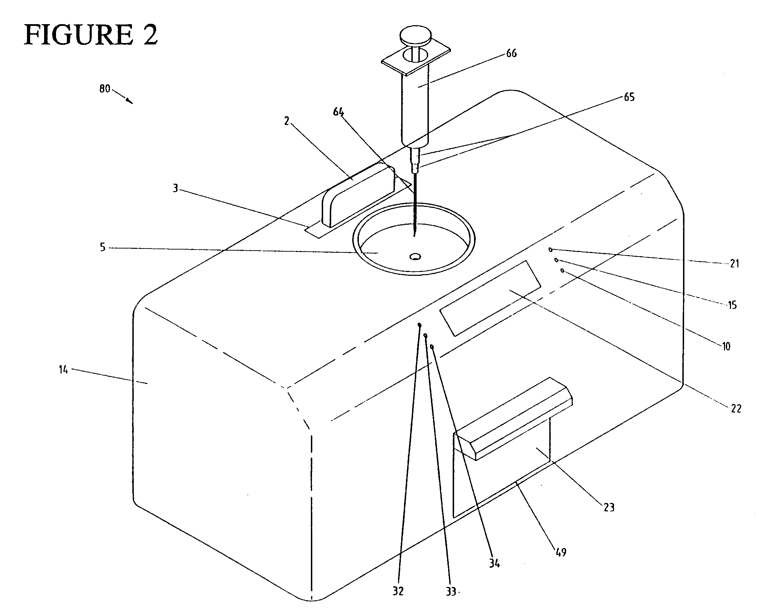

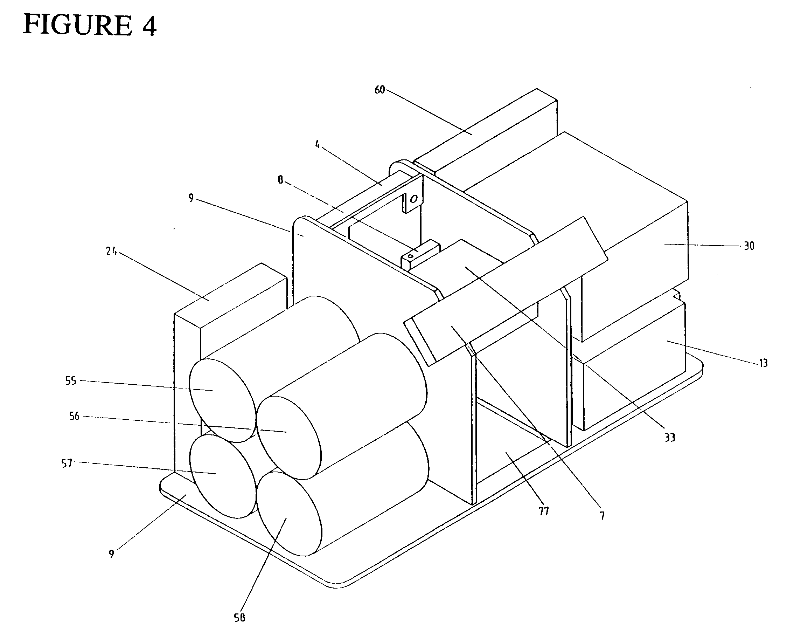

Referring to Figures 1 to 4 in particular, the incinerator comprises a housing 14 provided with a plunger 5 having a central aperture through which the needle to be incinerated is inserted. The plunger 5 moves downwardly relative to the remainder of the housing to trigger the needle incineration cycle as will be explained in more detail below. The housing 14 is provided with a tray receptacle 23 which fits into aperture 49 to collect the incinerated needle (in the form of ash) subsequent to the incineration cycle being completed.

Alternatively, where the waste container 162 is used tray 23 may be removed and the incinerated ash may fall directly into container 162.

Power means for the incinerator is provided either by rechargeable batteries 55,56,57 and 58, or a mains connectable electrical voltage step-down transformer 13 having a primary side of 110V or 220V/2.5A and a secondary side of 2.5V/120A. (These are mounted in the interior of housing 14 on support platform 9.)

Status lights 10,15,21,32,33,34, provided on the exterior of the housing enable an operative to ascertain when the incinerator is switched on, when the incineration cycle is in operation, when the incinerator is overheating, when the rechargeable batteries are in need of recharging, when the power is low and when the batteries are fully charged.

The incinerator is provided with a fume filtration system comprising an electrically powered fan 4 arranged to draw air (and fumes/dust) from the incineration zone through a filter board 2 (located in slot 3) to the atmosphere. The filter board 2 includes a filtration sheet 47 adapted to separate the fumes/dust particles from the air exiting to the atmosphere.

Additionally, the incinerator is provided with a liquid crystal display 22 to alternately indicate the quantity of needles incinerated since the rechargeable batteries 55,56,57,58 were last recharged and also indicate the quantity of needles incinerated since the incinerator unit was last serviced.

The incinerator is further provided with the appropriate microprocessor and/or integrated circuitry and thermistors to detect overheating, short circuits between the contact means and low battery power.

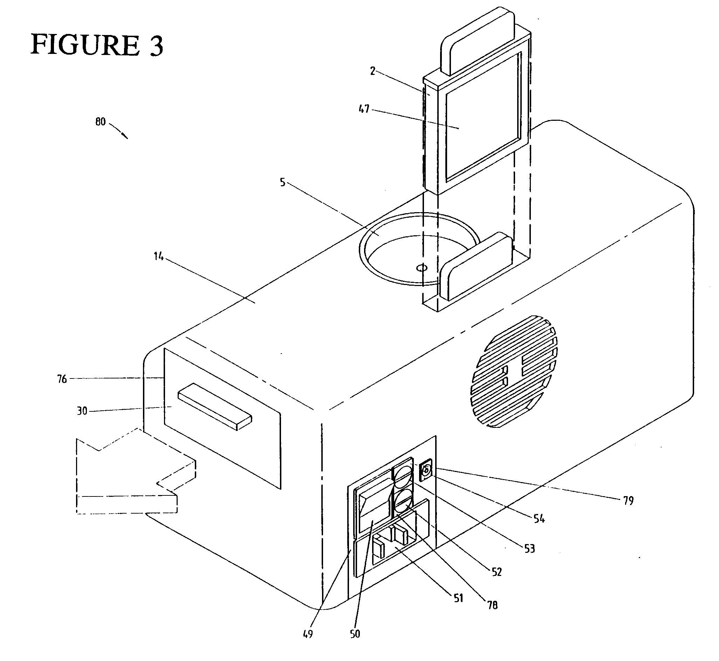

Referring to Figure 3, the control/interface panel on the rear of the housing can clearly be seen showing the mains plug socket 51, on/off switch 50 and fuses 52,53 and fuse holders 78,79. Also shown in Figure 3 is the incineration module 30 which is removable from the remainder of the incinerator housing, and described in greater detail below.

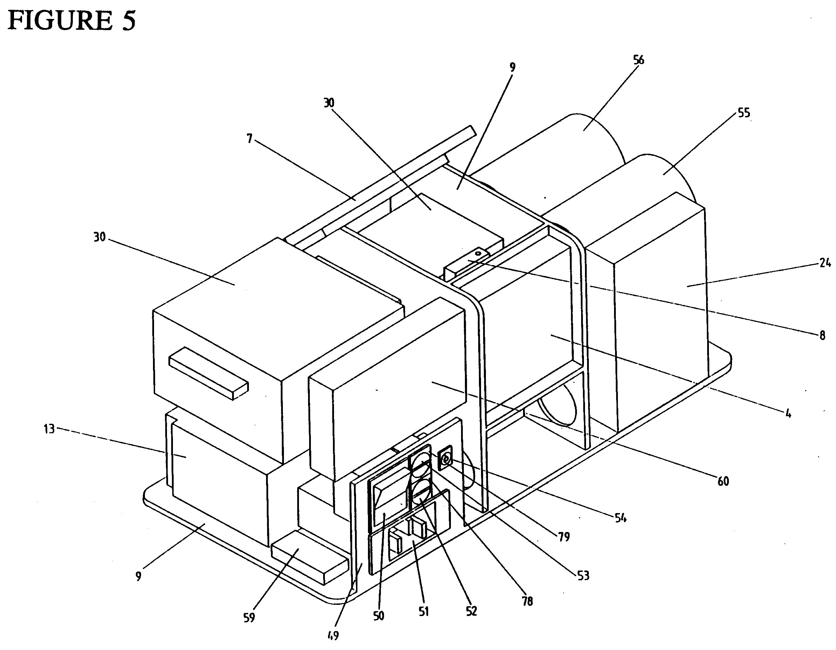

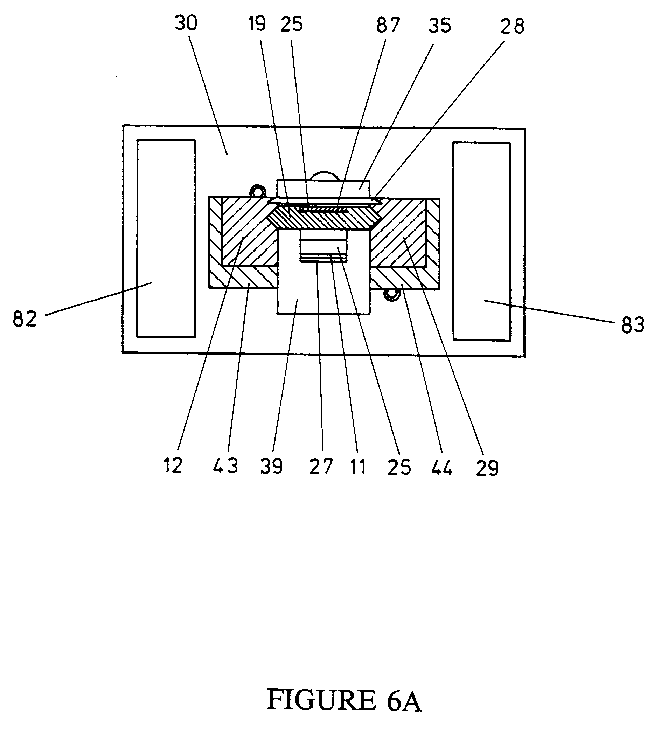

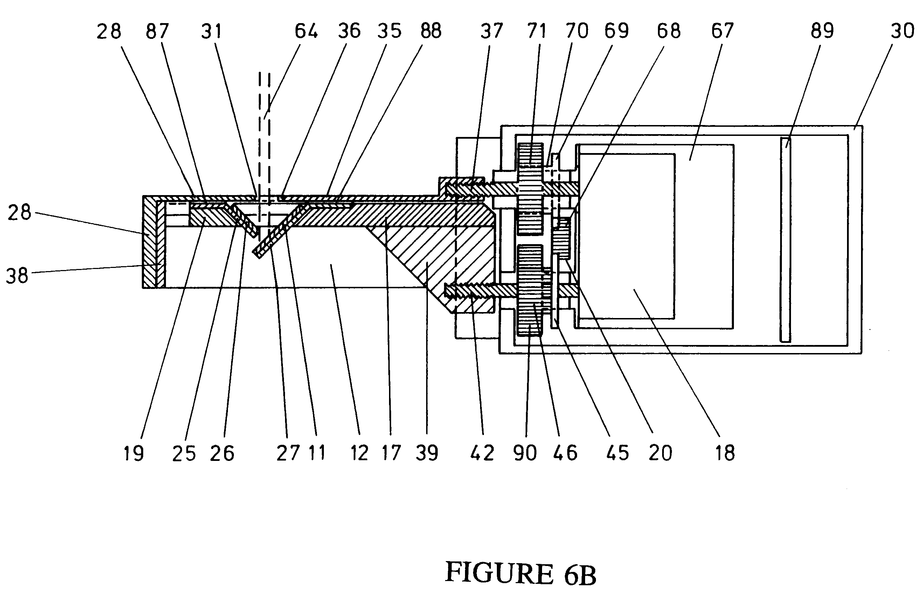

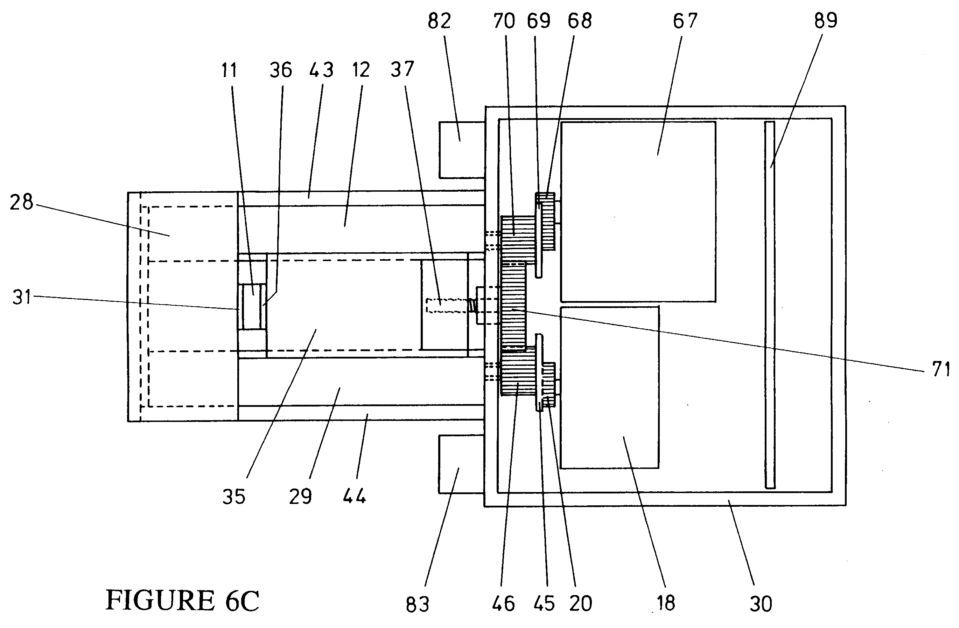

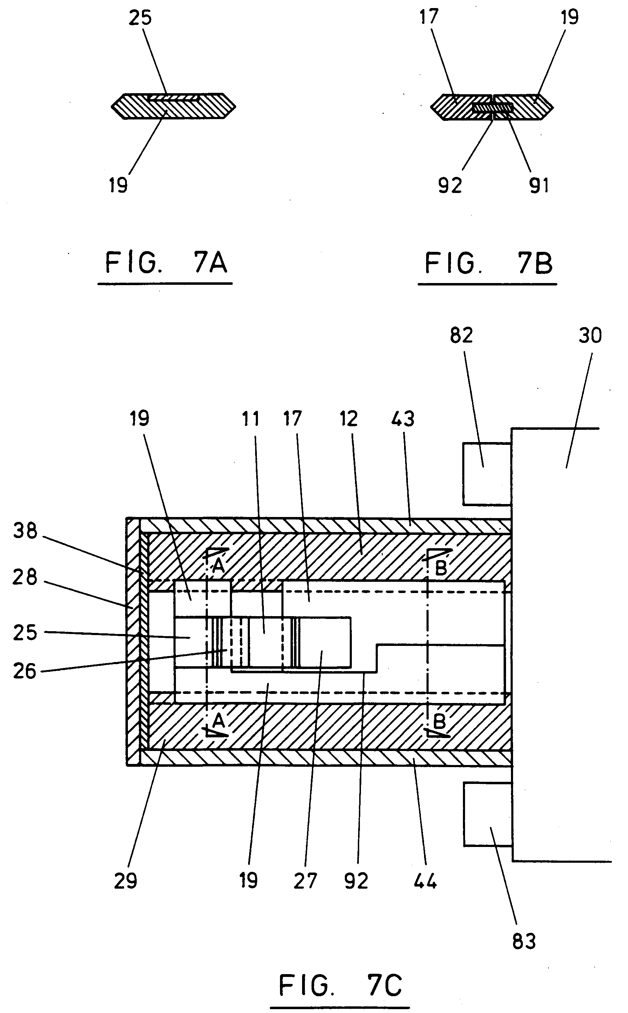

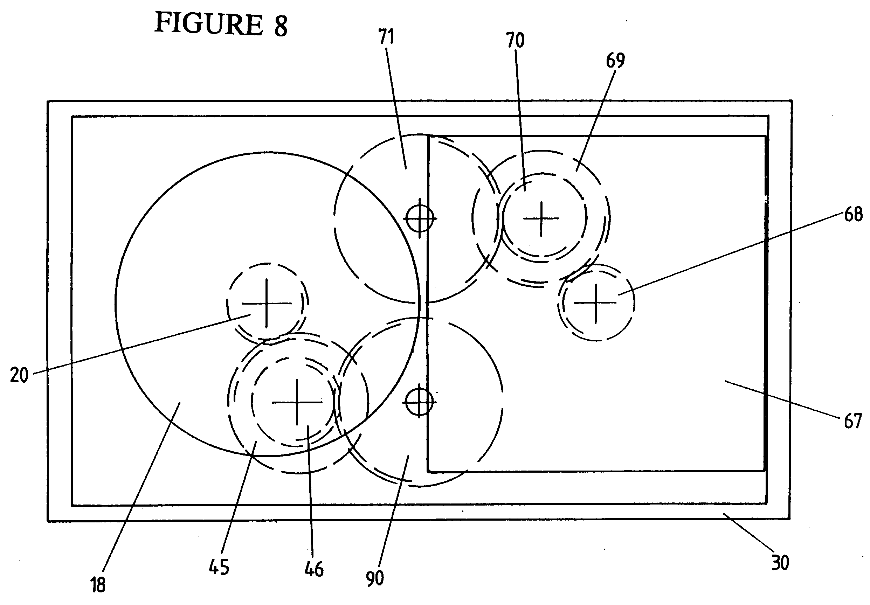

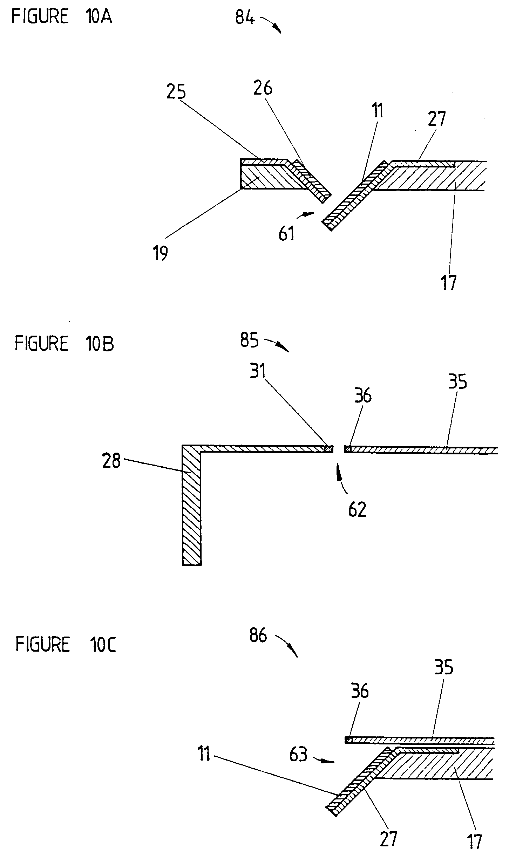

Referring to Figures 5 to 10C in particular, when the incinerator switch 50 is switched to the "ON" position appropriate microprocessor and/or integrated circuitry checks that no current is passing through the contact means 84,85,86 (Figures 10A to 10C) and checks the contacts 11,26,31 and 36 are correctly spaced for receiving a needle.

The incineration module 30 slides into the housing 14 (on guide rods not shown) and electrical contact is made with the power supply means by conductive contact pins (not shown), thereby setting up an electrical potential difference between incineration electrodes 25/26 and 27/11. The structural configuration of the incineration module may be best described with reference to its use below.

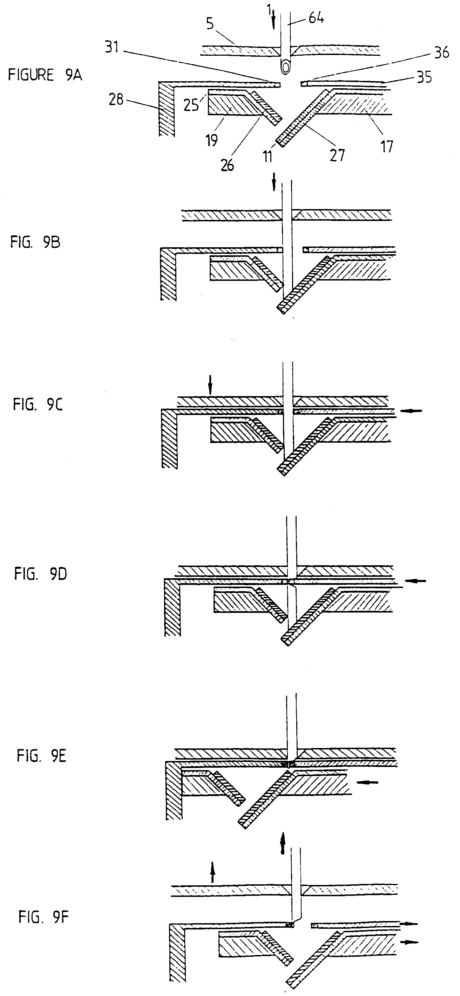

A needle 64 of a medical syringe 66 is inserted through the central aperture in the plunger 5. The needle 64 is pushed downwardly until the longitudinal side of the needle touches the incineration electrode 25/26 and the tip of the needle touches the incineration electrode 27/11. At this stage the contact means 84 (see Figure 10A) is completed between electrodes 25/26 and 27/11 across gap 61. By means of appropriate microprocessor and/or integrated circuitry a change in current supplied between the electrodes is detected and a sufficiently high current is passed between the contact electrodes (approximately 100A) such that the temperature of the needle between the contact electrodes is raised to between 800 and 1000 °C and incineration (or disintegration) of the needle between electrodes 25/26 and 27/11 is effected. This process continues (Figures 9A to 9F) as the needle is pushed down until eventually the needle hub 65 contacts the plunger 5. Since the needle hub 65 is of greater diameter than the aperture in plunger 5, continued downward pressure on syringe 66 causes the plunger 5 to travel downwardly causing the needle to continue its downward passage and incineration of the portions of the needle contacting and intermediate incineration electrodes 25/26 and 27/11 to continue as described above.

Eventually, the base of the plunger contacts and depresses the actuation button of a microswitch 8. The microswitch 8 is connected to microprocessor and/or integrated circuitry which completes a circuit to a stepping motor 67 housed in the incineration module 30. Stepping motor 67 drives a lead screw 37 (via gear train 68,69,70,71) which urges a movable section of module 30 towards a stationary section 12,28,29,31 of the module. The movable section comprises components 35,36. As the movable section nears the stationary section, the portion of the needle 64 immediately adjacent the hub 65 (which has not at this point bridged gap 61 and has therefore not been incinerated) is crimped between crimping contacts 31,36 (see Figure 10B) on the stationary and movable sections of housing 30 respectively. Since the crimping contacts 31,36 are connected to the power supply through the microprocessor and/or integrated circuitry which detects the diameter of the needle and supplies the appropriate and predetermined power supply to melt (incinerate) the needle, the microprocessor and/or integrated circuitry progressively supplies power to the motor (and thus urges the movable section) and also sufficient power to the contacts.

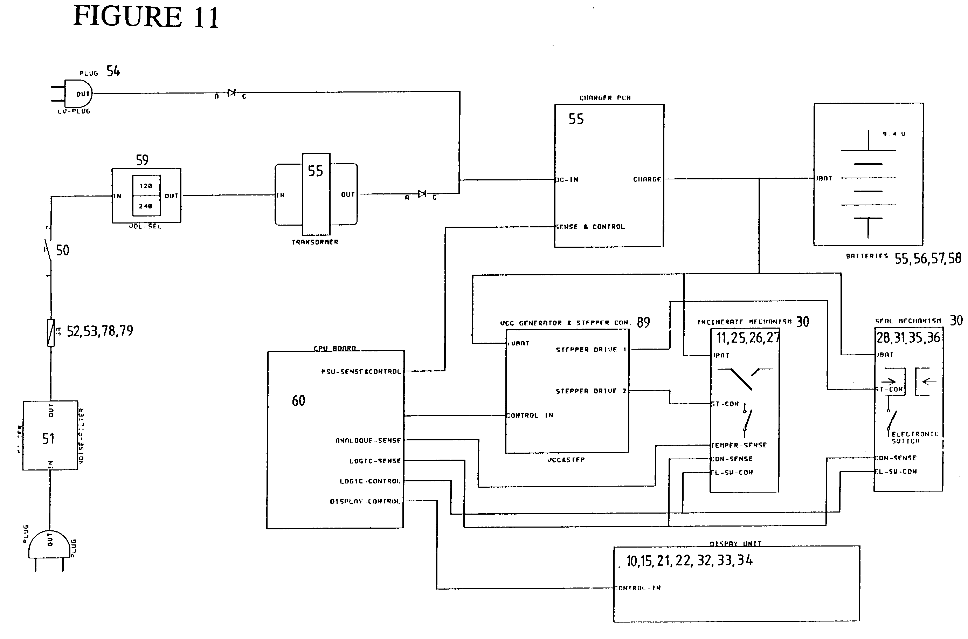

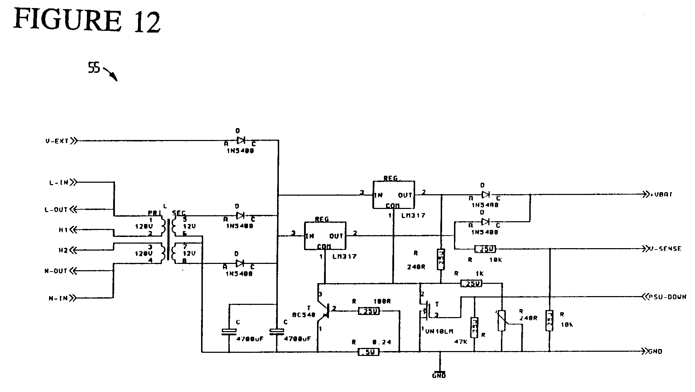

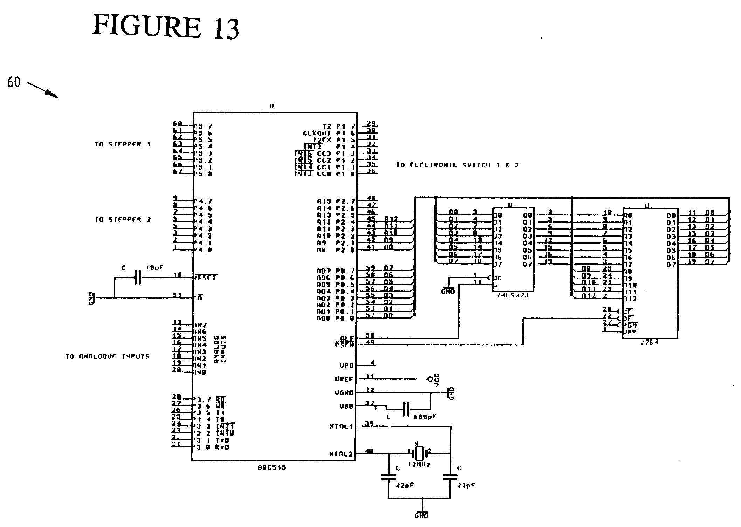

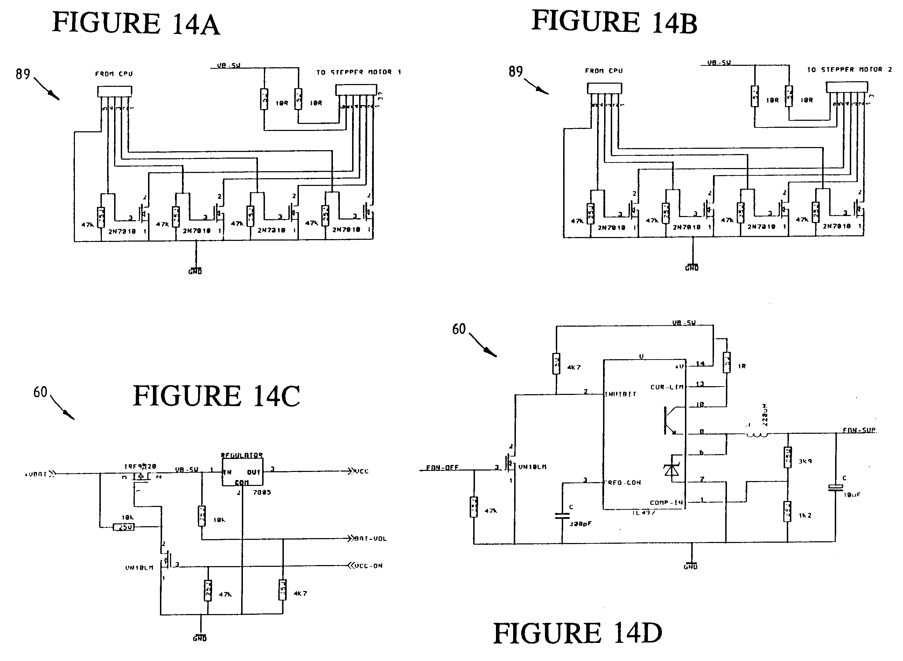

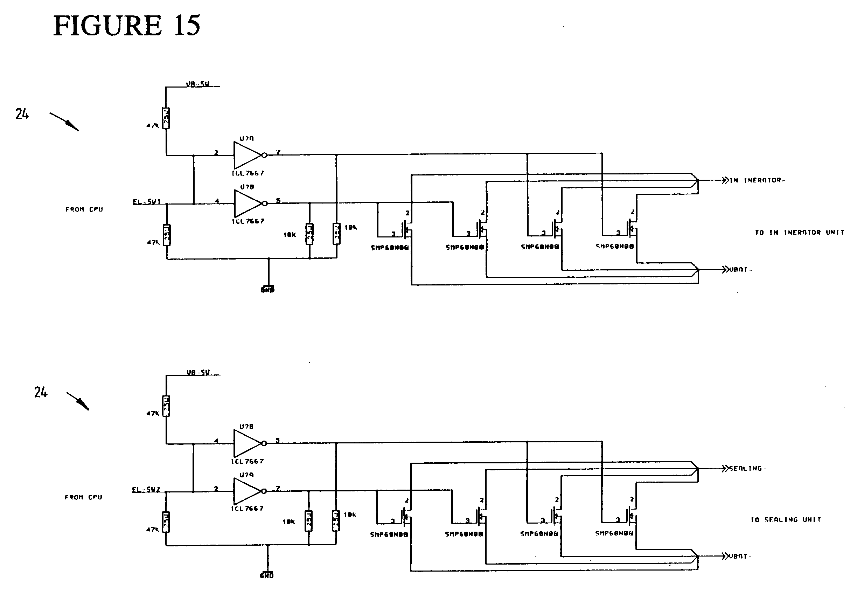

Once the needle is crimped between the contacts and the circuit between them across gap 62 is completed, the crimped end of the needle is incinerated, effectively ensuring sealing of the (now reduced) end of the needle and preventing possibly contaminating fluids escaping from the syringe 66. After the needle is crimped between the crimping contacts 31,36 the microprocessor and/or integrated circuitry switches on a sufficiently high power to a third circuit between contacts 11/27 and 36 across gap 63. The microprocessor and/or integrated circuitry also switches on the power supply to another electric stepping motor 18 which drives a lead screw 42 (via gear train 20,45,46,90) which urges a movable section of module 30. The movable section comprises 11,17,19,25,26,27,91,92. Thus the portion of the needle extending across the gap 63 (i.e. below the crimped portion of the needle) is therefore also incinerated (see Figure 10C). The microprocessor and/or integrated circuitry is set to reverse both the motors when the gap 62 between contact crimping electrodes 31 and 36 is approximately 0.05mm. Thus the movable sections (35,36 and 11,17,19,25,26,27,39,91,92) of incineration module 30 move back to their appropriate starting positions. The syringe can then be removed from the incinerator and the microprocessor and/or integrated circuitry resets the system ready for the next needle to be incinerated. The operation of the timing circuitry and motor/power switching is controlled by microprocessors provided on printed circuit board 60 contained within the incinerator housing 14 and on printed circuit board 60 contained within the incineration module 30. Figures 11 to 15 show circuit layouts and printed circuit board configurations of circuit boards 89,60,24,55 which comprise the controlling microprocessor/integrated circuitry referred to above.

Once a needle has been incinerated and crimped (i.e. once the incineration/crimping cycle is complete) the crimped needle 64 (still connected to syringe 66) is removed from the aperture in plunger 5, and the plunger 5 is reset to its starting position under the influence of four biasing springs (not shown).

The syringe 66 which now has only a very short portion of crimped and sealed needle 64 projecting therefrom may then be sent for disposal with the risk of potentially hazardous fluids escaping from the syringe and needlestick injury substantially reduced. The incinerated material from the needle (comprising of an ash or powder like material) is deposited in the waste tray 23 or a general medical waste container 162 under gravity.

Typically, the electrode (incineration) contacts 26,11,31,36 comprise 90% silver: 10% cadmium which has been found effective in inhibiting fusing (or welding) of the needle 64 to the contacts during incineration. A particularly advantageous feature of the incinerator according to the invention is the facility to completely remove the incinerator module 30 from the remainder of the incinerator for periodic overhaul or cleaning.

## Claims

1. An incinerator suitable for incinerating a syringe needle or other substantially hollow electrically-conductive article (64), the incinerator including a pair (11,26) of electrically-conductive contacts arranged to contact a portion of the article (64) at separate points along the length thereof to thermally degrade said portion when electricity is passed between the pair (11,26) of contacts through the article (64) to incinerate said portion of the article (64) characterised in that the incinerator comprises a second pair of electrically-conductive contacts (31,36) movable relative to one another under pressure into contact with a section of said article (64) at opposed positions across the width of the article so as to plastically deform said section of the article and to thermally degrade said section when electricity is passed between the second pair of contacts (31,36) through the article so as to seal off the hollow interior of the article.

2. An incinerator according to Claim 1, where said second pair of contacts (31,36) is spaced from said first pair of contacts (11,26) in the longitudinal direction of the article (64).

3. An incinerator according to Claim 1 or Claim 2, wherein one (26) of said first pair of contacts is arranged to contact the side of the article and the other (11) is arranged to contact the tip of the article.

4. An incinerator according to any one of Claims 1 to 3, comprising means by which a current may be caused to flow through the portion of the article between the first and second pairs of contacts causing that portion of the article to be thermally degraded.

5. An incinerator according to Claim 4, wherein said means comprises a third pair of contacts (36,11) which comprises one contact from each of said first and second pairs of contacts.

6. An incinerator according to any one of Claims 1 to 5, wherein said electrically-conductive contacts (11,26,31,36) are composed of 90% silver and 10% cadmium.

7. An incinerator according to any one of Claims 1 to 6, including electrical power supply means coupled to each pair of contacts (11,26;31,36;11,36) and arranged to produce an electrical potential difference across the respective gap between each pair of contacts.

8. An incinerator according to any preceding claim, wherein said pairs of contacts are positioned within a discrete module (30) removably separable from the remainder of the incinerator.

9. An incinerator according to Claim 7 in combination with claim 8, wherein said module (30) is provided with electrical supply coupling means arranged to cooperatively engage with complementary coupling means connected to said power supply means positioned in a housing (14) for the remainder of the incinerator, said module (30) being selectively removable from the housing (14) whereby said complementary coupling means are caused to disengage one another.

10. An incinerator according to any one of Claims 7 to 9, wherein the power supply means comprises at least one rechargeable battery (55,56,57,58) and the incinerator is configured to be portable.

11. An incinerator according to any one of Claims 7 to 10, wherein the electrical supply means is arranged to supply sufficient power to raise the temperature of said portion of the article to between 800 and 1000°C.

12. An incinerator according to any preceding claim, comprising movement means (67,37) causing said relative movement towards one another of said second pair of contacts, (31,36) and which is arranged to be actuated automatically at a predetermined stage in the incineration process.

13. An incinerator according to Claim 12, wherein said movement means (67,37) is adapted to be actuated when a predetermined length of said article has been inserted into the incinerator.

14. An incinerator according to Claim 12 or Claim 13, wherein insertion of said article into the incinerator causes a movable plunger (5) to complete a circuit when a predetermined plunger position is reached, completion of said circuit causing an electric motor (67) to actuate said movement means (37).

15. An incinerator according to Claim 14, wherein the circuit to said motor (67) includes a switch connected to reverse said motor after sealing of the article.

16. An incinerator according to any one of Claims 12 to 15, wherein the timing and duration of operation of said movement means is controlled by means of appropriate microprocessor and/or integrated circuitry.

17. An incinerator according to any preceding claim further provided with a removable receptacle (23) arranged to collect the incinerated portions of said article.

18. An incinerator according to any one of Claims 1 to 17, incorporated in a housing (14) enclosing said contacts (11,26,31,36), a filter (2), and a fan (4) positioned to move fumes from an incinerator zone through the filter (2) to exit said housing (14).

19. A method of disposing of a hypodermic needle (64) comprising the steps of inserting the needle (64) into an incinerator of the form as claimed in any one of Claims 1 to 18, contacting said portion of the length of the article with said first contacts (11,26) and passing an electric current through that portion to incinerate that portion, pressing said second contacts (31,36) into opposed sides of said section of the article, and passing an electric current through that section to effect sealing of the hollow interior of the needle.

20. A method according to Claim 19, wherein incineration of said portion of the needle is performed before sealing of said section.

21. A method according to Claim 20, wherein the needle is passed through a plunger (5) of the incinerator until the body (65) of the syringe contacts and presses down on the plunger (5), contacting the needle (64) by said first pair of contacts (11,26) and incinerating the portion of needle present between those contacts, continuing movement of the plunger (5) during such incineration until the plunger (5) reaches a position to operate a switch (8) to start a motor to cause movement of said second pair of contacts (31,36) into engagement with said section of the needle (64) to cause that section to be welded and sealed to prohibit the exit of any fluids through the needle from the syringe.

## Patentansprüche

1. Einäscherungsvorrichtung zum Einäschern einer Spritzennadel oder eines sonstigen, im wesentlichen hohlen, elektrisch leitfähigen Gegenstands (64), welche Einäscherungsvorrichtung ein Paar (11, 26) von elektrisch leitfähigen Kontakten aufweist, die zum Kontaktieren eines Abschnitts des Gegenstands (64) an über dessen Länge hinweg getrennten Stellen angeordnet sind, um den Gegenstand thermisch zu zersetzen (degrade), wenn Elektrizität zwischen dem Paar (11, 26) von Kontakten durch den Gegenstand (64) geleitet wird, um den genannten Abschnitt des Gegenstands (64) einzuäschern, dadurch gekennzeichnet, daß die Einäscherungsvorrichtung ein zweites Paar elektrisch leitfähiger Kontakte (31, 36) ausweist, die relativ zueinander unter Druck in Kontakt mit einer Sektion des Gegenstands (64) in einander gegenüberliegenden Positionen Über die Breite des Gegenstands bewegbar sind, um diese Sektion des Gegenstands plastisch zu deformieren und thermisch zu zersetzen, wenn Elektrizität zwischen dem zweiten Kontaktpaar (31, 36) durch den Gegenstand geleitet wird, um damit das hohle Innere des Gegenstands zu verschließen.

2. Einäscherungsvorrichtung nach Anspruch 1, wobei das zweite Kontaktpaar (31, 36) in der Längsrichtung des Gegenstands (64) vom ersten Kontaktpaar (11, 26) beabstandet ist.

3. Einäscherungsvorrichtung nach Anspruch 1 oder 2, wobei einer (26) der ersten beiden Kontakte zum Kontaktieren der Seite des Gegenstands und der andere (11) zum Kontaktieren der Spitze des Gegenstands angeordnet sind.

4. Einäscherungsvorrichtung nach einem der Ansprüche 1 bis 3, mit einem Mittel, mit dessen Hilfe ein Strom durch den Abschnitt des Gegenstands zwischen den ersten und zweiten Kontaktpaaren geleitet werden kann, um diesen Abschnitt des Gegenstands thermisch zu zersetzen.

5. Einäscherungsvorrichtung nach Anspruch 4, wobei das genannte Mittel ein drittes Paar von Kontakten (36, 11) aufweist, die jeweils einen Kontakt jedes der ersten und zweiten Kontaktpaare umfassen.

6. Einäscherungsvorrichtung nach einem der Ansprüche 1 bis 5, wobei die elektrisch leitfähigen Kontakte (11 26, 31, 36) aus 90% Silber und 10% Cadmium bestehen.

7. Einäscherungsvorrichtung nach einem der Ansprüche 1 bis 6, umfassend eine elektrische Stromversorgungseinrichtung, die an jedes Kontaktpaar (11, 26; 31, 36; 11, 36) angeschlossen und angeordnet ist, um eine elektrische Potentialdifferenz über den jeweiligen Spalt zwischen jedem Kontaktpaar zu erzeugen.

8. Einäscherungsvorrichtung nach einem der vorangehenden Ansprüche, wobei die Kontaktpaare in einem diskreten bzw. getrennten Baustein (30) positioniert sind, der vom Rest der Einäscherungsvorrichtung ausbaubar trennbar ist.

9. Einäscherungsvorrichtung nach Anspruch 7 in Kombination mit Anspruch 8, wobei der Baustein (30) mit (elektrischen) Stromversorgungsanschlußmitteln vorsehen ist, die angeordnet sind für zusammenwirkeende Verbindung mit komplementären Anschlußmitteln, die mit der in einem Gehäuse (14) für den Rest der Einäscherungsvorrichtung positionierten Stromversorgungseinrichtung verbunden sind, welcher Baustein (30) selektiv aus dem Gehäuse (14) ausbaubar ist, wodurch die komplementären Anschlußmittel voneinander getrennt werden.

10. Einäscherungsvorrichtung nach einem der Ansprüche 7 bis 9, wobei die Stromversorgungseinrichtung mindestens eine wiederaufladbare Batterie (55, 56, 57, 58) aufweist und die Einäscherungsvorrichtung so ausgestaltet ist, daß sie tragbar ist.

11. Einäscherungsvorrichtung nach einem der Ansprüche 7 bis 10, wobei die (elektrische) Stromversorgungseinrichtung angeordnet ist für Zuspeisung eines ansreichenden Stroms, um die Temperatur des (genannten) Abschnitts des Gegenstands auf eine Größe zwischen 800° und 1000°C zu erhöhen.

12. Einäscherungsvorrichtung nach einem der vorangehenden Ansprüche, umfassend ein Verschiebungsmittel (67, 37) zur Herbeiführung der Relativbewegung des zweiten Kontaktpaars (31, 36) aufeinander zu, welches Verschiebungsmittel angeordnet ist, um in einer vorbestimmten Stufe des Einäscherungsprozesses automatisch betätigt zu werden.

13. Einäscherungsvorrichtung nach Anspruch 12, wobei das Verschiebungsmittel (67, 37) betätigbar ist, wenn eine vorbestimmte Länge des Gegenstands in die Einäscherungsvorrichtung eingeführt worden ist.

14. Einäscherungsvorrichtung nach Anspruch 12 oder 13, wobei das Einführen des Gegenstands in die Einäscherungsvorrichtung bewirkt, daß ein verschiebbarer Stempel (5) beim Erreichen einer vorbestimmten Stempelstellung einen Stromkreis schließt, wobei beim Schließen des Stromkreises ein Elektromotor (67) zum Betätigen des Verschiebungsmittels (37) veranlaßt wird.

15. Einäscherungsvorrichtung nach Anspruch 14, wobei der Stromkreis zum Motor (67) einen Schalter beinhaltet, der zum Reversieren bzw. Umsteuern des Motors nach dem Verschließen des Gegenstands geschaltet ist.

16. Einäscherungsvorrichtung nach einem der Ansprüche 12 bis 15, wobei der Zeitpunkt und die Dauer der Betätigung des Verschiebungsmittels mittels eines (einer) zweckmäßigen Mikroprozessors und/oder integrierten Schaltung gesteuert werden.

17. Einäscherungsvorrichtung nach einem der vorangehenden Ansprüche, ferner versehen mit einem herausnehmbaren Aufnahmebehälter (23), der angeordnet ist zum Sammeln der eingeäscherten Abschnitte des Gegenstands.

18. Einäscherungsvorrichtung nach einem der Ansprüche 1 bis 17, integriert in ein die Kontakte (11, 26, 31, 36), ein Filter (2) und ein Gebläse (4), das angeordnet ist zum Austreiben von Abgasen aus einer Einäscherungszone durch das Filter (2) für Austritt aus dem Gehäuse (14), umschließendes Gehäuse (14).

19. Verfahren zum Entsorgen einer Subkutannadel (64), umfassend die Schritte eines Einführens der Nadel (64) in eine Einäscherungsvorrichtung der in einem der Ansprüche 1 bis 18 beanspruchten Form; Kontaktieren des Abschnitts der Länge des Gegenstands mit ersten Kontakten (11, 26) und Hindurchleiten eines elektrischen Stroms durch diesen Abschnitt, um ihn einzuäschern; Einpressen der zweiten Kontakte (31, 36) in gegenüberliegende Seiten der (genannten) Sektion des Gegenstands; und Leiten eines elektrischen Stroms durch diese Sektion, um ein verschließen des hohlen Inneren der Nadel herbeizuführen.

20. Verfahren nach Anspruch 19, wobei das Einäschern des (genannten) Abschnitts der Nadel vor dem Verschließen der (genannten) Sektion durchgeführt wird.

21. Verfahren nach Anspruch 20, wobei die Nadel durch einen Stempel (5) der Einäscherungsvorrichtung hindurchgeführt wird, bis der Körper (65) der Spritze den Stempel (5) kontaktiert und an ihm bzw. ihn herabdrückt, die Nadel (64) von dem ersten Kontaktpaar (11, 26) kontaktiert und der zwischen diesen Kontakten befindliche Abschnitt der Nadel eingeäschert wird, die Verschiebung des Stempels (5) während dieser Einäscherung fortgesetzt wird, bis der Stempel (5) eine Stellung zum Betätigen eines Schalters (8) zum Starten eines Motors zwecks Hervorbringung einer Verschiebung des zweiten Kontaktpaars (31, 36) in Anlage an die (genannte) Sektion der Nadel (64) erreicht, um diese Sektion zu verschweißen und zu verschließen und damit den Austritt etwaiger Fluide über die Nadel aus der Spritze zu verhindern.

## Revendications

1. Incinérateur approprié pour incinérer une aiguille de seringue ou un autre article électriquement conducteur substantiellement creux (64), l'incinérateur comprenant une paire (11, 26) de contacts électriquement conducteurs disposés pour venir au contact d'une partie de l'article (64) en des points séparés sur la longueur de celui-ci afin de dégrader thermiquement ladite partie lorsque de l'électricité passe entre la paire (11,26) de contacts en traversant l'article (64) pour incinérer ladite partie de l'article (64), caractérisé en ce que l'incinérateur comprend une deuxième paire de contacts électriquement conducteurs (31, 36) mobiles l'un par rapport à l'autre sous une pression pour être amenés au contact d'une section dudit article (64) en des positions opposées sur la largeur de l'article de manière à déformer plastiquement ladite section de l'article et à dégrader thermiquement ladite section lorsque de l'électricité passe entre la seconde paire de contacts (31, 36) en traversant l'article afin de sceller l'intérieur creux de l'article.

2. Incinérateur selon la revendication 1, dans lequel ladite deuxième paire de contacts (31, 36) est espacée de ladite première paire de contacts (11, 26) dans la direction longitudinale de l'article (64).

3. Incinérateur selon la revendication 1 ou la revendication 2, dans lequel l'un (26) des contacts de ladite première paire de contacts est disposé de manière à venir au contact du côté de l'article et l'autre contact (11) est disposé de manière à venir au contact de la pointe de l'article.

4. Incinérateur selon l'une quelconque des revendications 1 à 3, comprenant des moyens permettant de faire circuler un courant à travers la partie de l'article comprise entre la première et la deuxième paires de contacts pour dégrader thermiquement cette partie de l'article.

5. Incinérateur selon la revendication 4, dans lequel lesdits moyens comprennent une troisième paire de contacts (36, 11) qui se compose d'un contact de chacune desdites première et deuxième paires de contacts.

6. Incinérateur selon l'une quelconque des revendications 1 à 5, dans lequel lesdits contacts électriquement conducteurs (11, 26, 31, 36) sont composés de 90 % d'argent et 10 % de cadmium.

7. Incinérateur selon l'une quelconque des revendications 1 à 6, comprenant des moyens d'alimentation en courant électrique couplés à chaque paire de contacts (11, 26 ; 31, 36 ; 11, 36) et disposés pour produire une différence de potentiel électrique dans l'espace respectif séparant chaque paire de contacts.

8. Incinérateur selon l'une quelconque des revendications précédentes, dans lequel lesdites paires de contacts sont disposées dans un module discret (30) pouvant être séparé et retiré du reste de l'incinérateur.

9. Incinérateur selon la revendication 7 en combinaison avec la revendication 8, dans lequel ledit module (30) est équipé de moyens de connexion d'alimentation électrique disposés de manière à se mettre en prise et à coopérer avec des moyens de connexion complémentaires connectés auxdits moyens d'alimentation électrique dans un logement (14) pour le reste de l'incinérateur, ledit module (30) pouvant être retiré de manière sélective du logement (14) de manière à provoquer le désengagement desdits moyens de connexion complémentaires.

10. Incinérateur selon l'une quelconque des revendications 7 à 9, dans lequel les moyens d'alimentation comprennent au moins une batterie rechargeable (55, 56, 57, 58) et l'incinérateur est conçu pour être portatif.

11. Incinérateur selon l'une quelconque des revendications 7 à 10, dans lequel les moyens d'alimentation électrique sont disposés pour fournir une puissance suffisante pour élever la température de ladite partie de l'article à une température comprise entre 800 et 1000°C.

12. Incinérateur selon l'une quelconque des revendications précédentes, comprenant des moyens de déplacement (67, 37) provoquant le déplacement relatif des contacts de ladite deuxième paire de contacts (31, 36) l'un vers l'autre et qui sont disposés pour être actionnés automatiquement à une étape prédéterminée du processus d'incinération.

13. Incinérateur selon la revendication 12, dans lequel lesdits moyens de déplacement (67, 37) sont adaptés pour être actionnés lorsqu'une longueur prédéterminée dudit article est introduite dans l'incinérateur.

14. Incinérateur selon la revendication 12 ou la revendication 13, dans lequel l'introduction dudit article dans l'incinérateur amène un piston mobile (5) à fermer un circuit lorsque le piston atteint une position prédéterminée la fermeture dudit circuit amenant un moteur électrique (67) à actionner lesdits moyens de déplacement (37).

15. Incinérateur selon la revendication 14, dans lequel le circuit dudit moteur (67) comprend un commutateur connecté pour inverser ledit moteur une fois que l'article est scellé.

16. Incinérateur selon l'une quelconque des revendications 12 à 15, dans lequel l'instant et la durée de fonctionnement desdits moyens de déplacement sont commandés au moyen d'un microprocesseur et/ou circuit intégré appropriés.

17. Incinérateur selon l'une quelconque des revendications précédentes, équipé en outre d'un réceptacle amovible (23) disposé de manière à recueillir les parties incinérées dudit article.

18. Incinérateur selon l'une quelconque des revendications 1 à 17, disposé dans un logement (14) renfermant lesdits contacts (11, 26, 31, 36), un filtre (2) et un ventilateur (4) disposé pour faire passer les fumées d'une zone d'incinérateur à travers le filtre (2) vers l'extérieur dudit logement (14).

19. Procédé d'élimination d'une aiguille hypodermique (64) comprenant les étapes consistant à introduire l'aiguille (64) dans un incinérateur de la forme selon l'une quelconque des revendications 1 à 18, à mettre en contact ladite partie de la longueur de l'article et lesdits premiers contacts (11, 26) et à faire passer un courant électrique dans cette partie pour incinérer cette partie, à presser lesdits deuxièmes contacts (31, 36) contre des côtés opposés de ladite section de l'article, et à faire passer un courant électrique dans cette section pour réaliser le scellement de l'intérieur creux de l'aiguille.

20. Procédé selon la revendication 19, dans lequel l'incinération de ladite partie de l'aiguille est réalisée avant de sceller ladite section.

21. Procédé selon la revendication 20, dans lequel l'aiguille est introduite dans un piston (5) de l'incinérateur jusqu'à ce ce le corps (65) de la seringue touche et pousse le piston (5) vers le bas, ledit procédé conprenant les étapes consistant à mettre la seringue (64) en contact avec ladite première paire de contacts (11, 26) et à incinérer la partie de l'aiguille présente entre ces contacts, à poursuivre le déplacement du piston (5) pendant cette incinération jusqu'à ce que le piston (5) atteigne une position où il actionne un commutateur (8) faisant démarrer un moteur afin de déplacer ladite deuxième paire de contacts (31, 36) en prise avec ladite section de l'aiguille (64), de manière à ce que cette section soit soudée et scellée pour empêcher que des fluides quels qu'ils soient s'échappent de la seringue à travers l'aiguille.
